Europäisches Patentamt

European Patent Office

Office européen des brevets

(19)

(11) Publication number: **0 148 587**
A2

# EUROPEAN PATENT APPLICATION

(21) Application number: **84308416.1**

(22) Date of filing: **04.12.84**

(51) Int. Cl.⁴: **A 41 B 13/02**

(30) Priority: **06.12.83 US 558449**

(43) Date of publication of application: **17.07.85**
**Bulletin 85/29**

(84) Designated Contracting States: **BE CH DE FR GB IT LI SE**

(71) Applicant: **MINNESOTA MINING AND MANUFACTURING COMPANY, 3M Center, P.O. Box 33427, St. Paul, MN 55133 (US)**

(72) Inventor: **Pape, Peter H.K. c/o Minnesota Mining and, Manufacturing Co. 2501 Hudson Road P.O. Box 33427, St. Paul Minnesota 55133-3427 (US)**

(74) Representative: **Baillie, Iain Cameron et al, c/o Ladas & Parry Isartorplatz 5, D-8000 München 2 (DE)**

(54) **Refastenable tape closure for disposable diapers and composite tape for the preparation of such closures.**

(57) Composite roll of tape especially suitable for making a refastenable tape closure for disposable diapers. The construction involves a pressure-sensitive adhesive fastening tape that partially overlies a pressure-sensitive adhesive target tape and also partially overlies a pressure-sensitive adhesive release tape, adhesion to the target tape preferably substantially exceeding the adhesion to the release tape. In use, the target tape and the release tape bond permanently to opposite sides of one edge of the diaper, one end of the fastening tape being thereafter lifted free from the release tape and permanently adhered to the opposite edge of the diaper. The opposite end of the fastening tape can then be repeatedly removed and re-adhered to the surface of the target tape.

-1-

## REFASTENABLE TAPE CLOSURE FOR DISPOSABLE DIAPERS AND COMPOSITE TAPE FOR THE PREPARATION OF SUCH CLOSURES

### Technical Field

This invention relates to disposable diapers having normally tacky and pressure-sensitive adhesive tape closures and is particularly concerned with composite closures (i.e., closures made with more than one tape) that can be opened and refastened without destroying either the diaper or the tape.

### Background Art

At least as early as 1955 it had been suggested to use strips of normally tacky and pressure-sensitive adhesive tape to hold conventional cloth diapers on an infant; see, e.g., Chambers U.S. Pat. No. 2,714,889 and Ekberg U.S. Pat. No. 3,221,738. A few years later, when disposable diapers became extremely popular, strips of pressure-sensitive adhesive tape were again employed as closures; see, e.g., Gellert U.S. Pat. No. 3,620,217.

A disposable diaper typically has a thin, flexible, stretchy low density polyethylene film cover, an absorbent filler on the inside of the cover, and a porous inner liner overlying the filler. Such a diaper is positioned at the crotch of an infant, the two ends of the diaper extending, respectively, toward the front and back. Adjacent edges of the diaper at each side are then either positioned next to each other or overlapped, a strip of pressure-sensitive adhesive tape being adhered to the cover at the border adjacent each of the two edges and holding the diaper closed. Because the pressure-sensitive adhesive bonds firmly to the thin polyethylene diaper cover, it has heretofore been essentially impossible to open the tape closure without destroying the tape, the diaper cover, or both in the process.

-2-

After a tape closure has been opened, it is frequently discovered that the diaper has not been soiled and hence that there is no need to replace it. If the cover has not been torn, a second strip of tape can sometimes be applied as a replacement closure, but this is often inconvenient. As a result, considerable work has been undertaken to develop a tape diaper closure that is not only capable of bonding firmly to the diaper cover, but is also capable of non-destructive removal and replacement. Closures of this type have generally involved a combination of two or more tapes, one of which remains permanently adhered to one edge of the diaper and is removably adhered to a so-called "target tape" mounted on the other edge of the diaper. Examples of such products are shown in Ness et al, U.S. Pat. No. 3,951,149, Milnamow U.S. Pat. No. 3,987,793, Feldman et al U.S. Pat. No. 3,999,546, and Richman et al U.S. Pat. No. 4,020,842.

The patents referred to in the preceding paragraph do not discuss the manner in which the closures are prepared. Typically, however, the manufacturer of diapers mounts rolls of the appropriate tape in his equipment, combining them to form a composite strip of tape, the width of which is substantially the same as the length of the diaper closure to be fabricated. The composite roll is then severed at right angles to the edges of the composite strip at intervals corresponding to the width of the desired tape closure and adhered at an appropriate location along the border adjacent the sides of the diaper. Although this manufacturing process is effective, many relatively unsophisticated or small manufacturers are unable to provide the machinery necessary to accomplish the superimposition of several rolls of tape; as a result, it is important for a tape supplier to provide such manufacturers with a composite roll, made up of two or more specific tapes from which closures may readily be prepared.

-3-

## Disclosure of Invention

The present invention provides a novel composite tape closure for disposable diapers of the type comprising a body of fluid-absorbing material having a fluid-impermeable polymeric foil outer cover, a pressure-sensitive adhesive tape closure permanently mounted at a first border location adjacent one edge of the diaper and adapted for attachment to the second border location adjacent the edge when the two locations are juxtaposed or overlapped. In accordance with the invention, the tape closure comprises a composite of

(a) a pressure-sensitive adhesive release tape adhered to the inner aspect of the diaper at the first location,

(b) a pressure-sensitive adhesive target tape adhered to the outer aspect of the diaper at the first location, and

(c) a pressure-sensitive adhesive fastening tape overlying and adhered to the backs of both the target tape and the release tape, the adhesion of the fastening tape to the back of the target tape being substantially less than the adhesion of the target tape to the diaper cover.

This composite permits the portion of the fastening tape adhered to the release tape to be lifted therefrom and adhered permanently to the outer aspect of the diaper at the second location, after which the portion of the fastening tape adhered to the target tape can be repeatedly lifted therefrom and re-adhered thereto.

Composite closures of the type just described are advantageously prepared from a roll of tape comprising a composite elongate strip of pressure-sensitive adhesive sheet material wound convolutely upon itself about an annular core. This composite strip is especially suited for preparing tape closures of the type described by simply

-4-

severing the elongate strip of tape at right angles to the axis of the core at intervals corresponding to the predetermined width of the closure. The composite strip comprises in combination:

    (a)  a fastening tape comprising an elongate strip of sheet backing material, having first and second edges, substantially as wide as the composite strip, and having a layer of normally tacky and pressure-sensitive adhesive coated over at least substantially the entire width of the backing material,

    (b)  the adhesive of the fastening tape adjacent the first edge thereof contacting and adhered to the back of a pressure-sensitive adhesive target tape, and

    (c)  the adhesive of the fastening tape adjacent the second edge thereof contacting and adhered to the back of the target tape than to the back of the release tape.

### Brief Description of the Drawing

The invention can be more easily understood by referring to the accompanying drawings, in which certain dimensions are exaggerated to facilitate understanding. Like numbers refer to like parts in the several views, and

FIGURE 1 shows a roll of composite tape suitable for use in practicing the invention;

FIGURE 2 is an enlarged cross-sectional view of the composite tape of Figure 1;

FIGURE 3 is an enlarged cross-sectional view of a first alternate embodiment of the composite tape of the invention;

FIGURE 4 is an enlarged cross-sectional view of a second alternate embodiment of the composite tape of the invention;

-5-

FIGURE 5 is a cross-sectional view showing juxtaposed diaper edges, a closure formed from the tape of Figures 1 and 2 being applied to one of the edges, the arrow indicating the manner in which the closure is moved for attachment to the second edge;

FIGURE 6 shows the tape closure resulting from carrying out the attachment step indicated in Figure 5; and

FIGURE 7 shows how the tape closure is opened without destroying either the tape or the diaper.

## Detailed Description

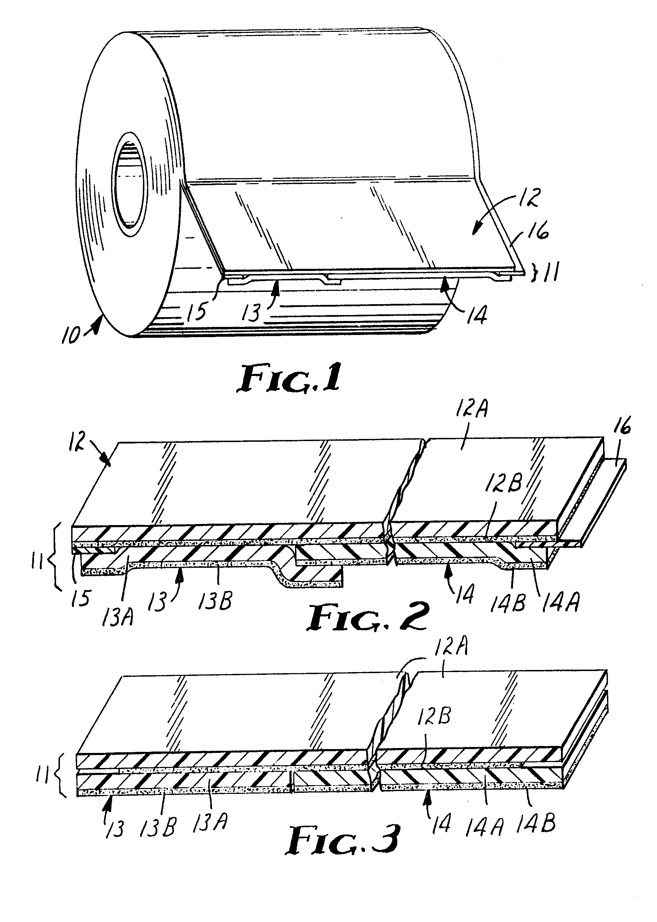

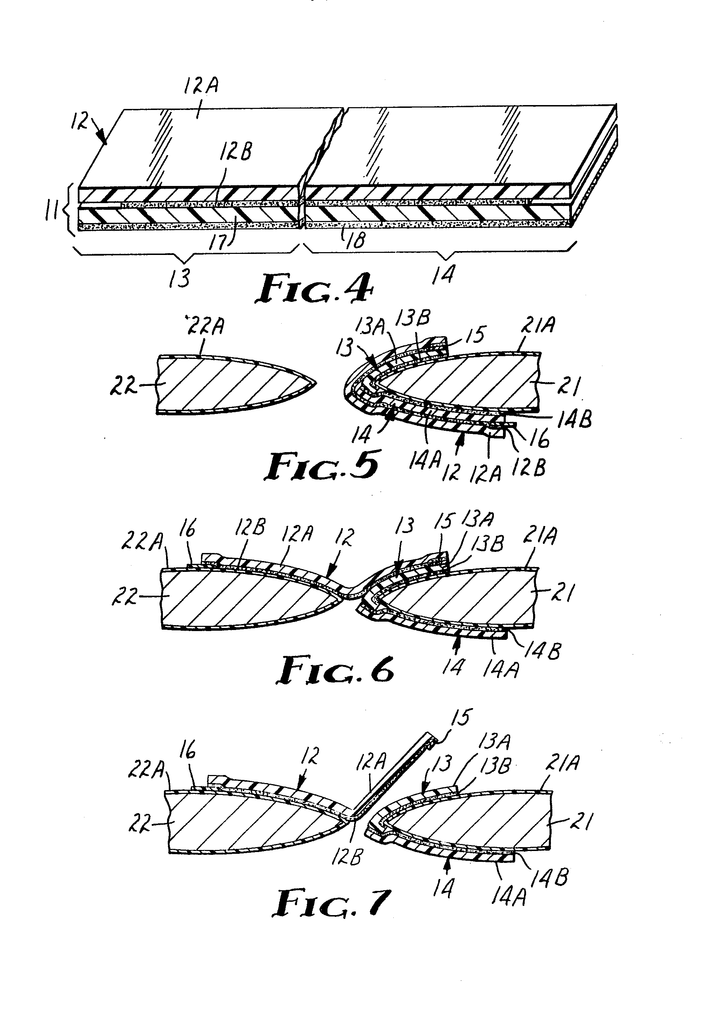

Turning first to FIGURES 1 and 2, tape roll 10 is formed of composite tape 11 wound convolutely upon itself about a core. Composite tape 11 is in turn made up of fastening tape 12, target tape 13, and release tape 14. Fastening tape 12 comprises any suitable tape backing 12a (e.g., treated creped paper, polymeric film, etc.) on one face of which is coated a layer of normally_tacky and pressure-sensitive adhesive 12b, typically of the so-called "rubber-resin" type. Target tape 13, which is narrower than fastening tape 12, is so positioned that it coincides with or approaches one end of the latter, adhesive 12b being adhered to the back of target tape backing 13a. A first tab 15, which is typically formed of a narrow strip of any suitable polymeric film, is interposed between backing 13a and pressure-sensitive adhesive 12b at one edge, facilitating the manual separation of tapes 12 and 13, as will be described subsequently in more detail.

At the opposite edge of composite tape strip 11 is release tape 14, again comprising conventional backing 14a and pressure-sensitive adhesive 14b. Interposed between pressure-sensitive adhesive 12b and release tape backing 14a is a second tab 16, generally similar to the one interposed between pressure-sensitive adhesive 12b and target tape backing 13a. For reasons to be explained subsequently in more detail in connection with the operation of tape closures, the back surface of release

tape backing 14a is typically provided with a conventional low adhesion backsize (LAB) to facilitate separation of tapes 12 and 14.

The construction of composite tape 11 shown in Figure 3 is essentially the same as that shown in Figure 1, but is somewhat simpler. Pressure-sensitive adhesive 12b does not extend completely to either edge of composite tape 11, the resultant lack of adhesion permitting the insertion of a fingernail to effect separation when desired. Additionally, release tape 14 does not overlap target tape 13, but instead abuts it.

The embodiment shown in Figure 4 is substantially the same as that of Figure 3, except that common backing 17 and common adhesive 18 are incorporated into a structure performing the dual functions of target tape 13 and release tape 14. This dual functionality is achieved by the application of an LAB to the right hand portion of common backing 17 but not to the left. It would, however, be possible to apply a single LAB to the entire back surface of backing 17, provided the force required to remove adhesive 12b from the backing 17 is comparable to that required to remove adhesive 12b from the back of target tape 13. Indeed, such a construction requiring only two relatively simple tapes, would be easy for either a tape manufacturer or a diaper manufacturer to prepare. In this product, it will be noted that there is no structural difference between target tape 13 and release tape 14.

The constructions shown in FIGURES 2 and 3 do, of course, lend themselves to the preparation of carefully tailored products having somewhat more sophisticated control than the construction shown in FIGURE 3.

An advantage of the embodiments shown in both Figures 3 and 4 is a substantially uniform side-to-side thickness of composite tape 11, facilitating smooth winding and uniform roll thickness. On the other hand, the structure depicted in Figure 1 requires somewhat less precision in relationship of the various tapes, is accordingly easier to manufacture, and the preparation of a satisfactory composite roll of tape is comparatively simple.

As previously indicated, Figures 5-7 illustrate the use of closures formed by severing composite tape 11 at intervals corresponding to the predetermined width of the closure, at right angles to the axis of the tape core. Thus, in Figure 5, diaper edges 21 and 22 are juxtaposed, surfaces 21a and 22a corresponding to the outer surface of the diaper cover, conventionally made of low density polyethylene foil. As shown in Figure 5, adhesive 13b of target tape 13 is adhered to diaper cover 21a in the area immediately adjacent the edge, extending slightly around the edge to the interior surface of the diaper. Similarly, release tape 14 is permanently adhered to the inner surface of diaper edge 21. Overlying and adhered to the back surfaces of both backing 13a of target tape 13 and backing 14a of release tape 14 is fastening tape 12. Because of the presence of an LAB on the back surface of release tape 14a, however, fastening tape 12 can be conveniently removed by grasping tab 16, moving one end of fastening tape 12 through the arc shown by the arrow, and then adhering it permanently to the polyethylene cover 22a of diaper edge 22, yielding the arrangement shown in Figure 6.

When it is desired to open the diaper closure, the opposite end of fastening tape 12 is lifted free of target tape 13, to which it bonds firmly enough to prevent inadvertent opening of the closure but not so firmly that it cannot be lifted free without tearing either backing 13a or polyethylene diaper cover 21a. Once lifted free, this end of fastening tape 12 can again be resealed by placing it in contact with the back surface 13a of target tape 13; indeed, the process can be successfully repeated several times.

-8-

CLAIMS:

1. A disposable diaper comprising a body of fluid-absorbent material having a fluid-impermeable polymeric foil outer cover, a pressure-sensitive adhesive tape closure permanently mounted at a first border location adjacent one edge of said diaper and adapted for attachment to a second border location adjacent said edge when the two locations are juxtaposed or overlapped, characterized in that said tape closure comprises a composite of

(a) a pressure-sensitive adhesive release tape adhered to the inner aspect of the diaper at said first location,

(b) a pressure-sensitive adhesive target tape adhered to the outer aspect of the diaper at said first location, and

(c) a pressure-sensitive adhesive fastening tape overlying and adhered to the backs of both the target tape and the release tape, the adhesion of the fastening tape to the back of the target tape being substantially less than the adhesion of the target tape to the diaper cover,

whereby the portion of the fastening tape adhered to the release tape can be lifted therefrom and adhered permanently to the outer aspect of the diaper at said second location, after which the portion of the fastening tape adhered to the target tape can be repeatedly lifted therefrom and re-adhered thereto.

2. The product of claim 1 further characterized in that the adhesion of the fastening tape to the back of the target tape substantially exceeds the adhesion of the target tape to the back of the release tape.

3. The product of claim 1 further characterized in that the end of the release tape nearer the edge of the diaper overlaps and is adhered to the end of the target tape nearer the edge of the diaper.

4. The product of claim 1 further characterized in that the release tape and the target tape have a common backing, with different low adhesion backsize on their respective backing portions.

5. The product of claim 1 further characterized in that the distal portions of the fastening tape are not adhered to either the release tape or the target tape.

6. The product of claim 5 further characterized in that a protective polymeric foil covers the adhesive of the fastening tape in said distal portions.

7. The product of claim 5 further characterized in that said distal portions are free from adhesive.

8. A roll of tape comprising a composite elongate strip of pressure-sensitive adhesive sheet material wound convolutely upon itself about an annular core, especially suited for preparing tape strips to make the tape closure of claim 1 by simply severing said elongate strip of tape at right angles to the axis of the core at intervals corresponding to the predetermined width of said closure, the length of each such closure corresponding to the width of the roll of tape, said composite elongate strip of tape characterized by comprising in combination

    (a)   a fastening tape comprising an elongate strip of sheet backing material, having first and second edges, being substantially as wide as said composite strip, and having a layer or normally tacky and pressure-sensitive adhesive coated over at least

-10-

substantially the entire width of said backing material,

(b) the adhesive of said fastening tape adjacent the first edge thereof contacting and adhered to the back of a pressure-sensitive adhesive target tape, and

(c) the adhesive of said fastening tape adjacent the second edge thereof contacting and adhered to the back of a pressure-sensitive adhesive release tape.

9. The roll of tape of claim 8 further characterized in that the adhesion of the fastening tape to the back of the target tape exceeds the adhesion of the fastening tape to the back of the release tape.

0148587

FIG.1

FIG. 2

FIG. 3

0148587

**FIG. 4**

**FIG. 5**

**FIG. 6**

**FIG. 7**